# EUROPEAN PATENT APPLICATION

(11) **EP 3 868 869 A1**
(43) Date of publication of application: **25.08.2021**
(21) Application number: 19873149.9
(22) Date of filing: 11.10.2019
(51) Int. Cl.: C12N 5/071, A23L 33/175, A61K 31/198, A61K 31/405, A61K 31/4172, A61P 3/02, A61P 43/00, C12N 5/0735, C12N 5/074

(54) **NUTRITION COMPOSITION**

(30) Priority: 15.10.2018 JP 2018194380
(71) Applicant: Public University Corporation Yokohama City University, Yokohama-shi, Kanagawa 236-0027 (JP); Takeda Pharmaceutical Company Limited, Osaka-shi, Osaka 541-0045 (JP)
(72) Inventor: TAKEBE, Takanori, Tokyo 113-8510 (JP); NIO, Yasunori, Fujisawa-shi, Kanagawa 251-0012 (JP)
(74) Representative: Jones, Nicholas Andrew
(86) International application number: PCT/JP2019/040145
(87) International publication number: WO 2020/080270

(57) **Abstract**

The present invention provides a means for suppressing formation and/or proliferation of undesired cells derived from stem cells in a cell population containing cells differentiated from stem cells. The nutrition composition according to the present invention is a nutrition composition for suppressing formation and/or proliferation of undesired cells derived from stem cells in a cell population containing cells differentiated from stem cells, the nutrition composition containing at least one essential amino acid selected from the group consisting of isoleucine, leucine, methionine, lysine, phenylalanine, tryptophan, threonine and histidine except valine, and optionally containing a non-essential amino acid(s).

## Description

### Technical Field

The present invention relates to a nutrition composition comprising predetermined essential amino acids and optionally a non-essential amino acid(s), and use of the nutrition composition. The present invention also relates to a means for suppressing formation and/or proliferation of undesired cells derived from stem cells in a cell population containing cells differentiated from stem cells such as iPS cells (induced pluripotent stem cells), *in vitro* or *in vivo.*

### Background art

In the cell therapy and regenerative medicine, stem cells such as iPS cells are differentiation-induced *in vitro* into desired cells or a cell population (tissue) containing the desired cells, and then, the desired cells or cell population are transplanted or administered for treating diseases or regenerating a diseased tissue. However, if undifferentiated stem cells (e.g., iPS cells) and cells (e.g., endoderm, mesoderm, ectoderm) that failed to differentiate into desired cells remain in the cells or cell population to be transplanted, there are risks of formation of teratoma and proliferation of cells that failed to differentiate into desired cells *in vivo* after transplantation. To prevent such risk events from the cell population to be used for transplantation and the like, it is necessary (1) to prevent, in the stage of obtaining a cell population by culturing before transplantation, remaining of undifferentiated stem cells and formation/remaining of cells that failed to differentiate into desired cells from stem cells, as much as possible, and (2) to suppress, in a cell population transplanted, formation of teratoma from undifferentiated stem cells and proliferation of cells that failed to differentiate into desired cells. In the cell population containing cells differentiated from stem cells, it is important to suppress formation and/or proliferation of undesired cells derived from stem cells in order to improve safety and effectiveness of cell therapy and regenerative medicine.

Non Patent Literature 1 discloses a methionine deficient diet (vegan diet for humans) for suppressing growth of cancer. Non Patent Literature 2 discloses that a serine and glycine deficient diet delays tumor growth in cancer-bearing rats having HCT116 (invasive human colonic rectal cancer cell strain) and declines *in-vivo* proliferation of HCT116 cells. Non Patent Literature 3 discloses autophagy dependent cell-death of argininosuccinate synthetase 1 (ASS1)-deficient breast cancer by arginine starvation. Non Patent Literature 4 suggests a therapeutic effect of arginine and glutamine starvation on various diseases including cancer. Non Patent Literature 5 discloses anti-tumor effects by enzymes causing starvation of asparagine, glutamine, methionine and the like. Non Patent Literature 6 discloses that tumor growth was suppressed in cancer-bearing rats by a methionine and valine deficient diet. Non Patent Literature 7 discloses regression of a tumor in cancer-bearing rats by a valine deficient diet.

However, Non Patent Literatures 1 to 7 all relate to a therapeutic effect of cancer (malignant tumor) and do not disclose that a nutrition composition lacking a predetermined amino acid and disclosed in each literature, can suppress formation and/or proliferation of undesired cells derived from stem cells in a cell population containing cells differentiated from stem cells.

Patent Literature 1 discloses a nutrition composition, e.g., for treatment of inflammatory diseases (e.g., inflammatory bowel disease such as Crohn's disease and ulcerative colitis), the nutrition composition comprising an amino acid composition consisting of all essential amino acids (histidine, isoleucine, leucine, lysine, methionine, phenylalanine, threonine, tryptophan, valine, cysteine and tyrosine) for humans, and comprising no non-essential amino acids for humans except arginine.

However, Patent Literature 1 does not disclose that a nutrition composition comprising an amino acid composition as mentioned above can suppress formation and/or proliferation of undesired cells derived from stem cells in a cell population containing cells differentiated from stem cells.

### Citation List

### Patent Literature

Patent Literature 1: JP 2012-201625 A (JP 5837315 B) .

### Non Patent Literatures

Non Patent Literature 1: Cancer Treatment Reviews 38 (2012) 726-736.
Non Patent Literature 2: Nature 544 (2017) 372-376.
Non Patent Literature 3: Sci. Signal 7 (391) pp. ra31.
Non Patent Literature 4: Nutrition in Clinical Practice 32 (Suppl 1) 2017 30S-47S.
Non Patent Literature 5: Cancer 43: 2137-2142, 1979.
Non Patent Literature 6: World J Gastroenterol 2003; 9 (12): 2772-2775.
Non Patent Literature 7: Tohoku J. exp. Med., 1988, 156, 259-270.

### Summary of Invention

### Technical Problem

An object of the present invention is to provide a means for suppressing formation and/or proliferation of undesired cells derived from stem cells in a cell population containing cells differentiated from stem cells, for use in, e.g., cell therapy and regenerative medicine, in a stage of producing desired cells and/or after transplantation and administration thereof to a living body.

### Solution to Problem

The present inventors have conducted intensive studies and have found that the relationship between cancer cells and intake of amino acids is not always in consistent with the relationship of undesired cells derived from stem cells and intake of amino acids. Based on the finding, the present invention has been accomplished.

In order to the above problem, the present invention provides the following [1] to [11]:
[1] A nutrition composition for suppressing formation and/or proliferation of undesired cells derived from stem cells in a cell population containing cells differentiated from stem cells, the nutrition composition comprising at least one essential amino acid selected from the group consisting of isoleucine, leucine, methionine, lysine, phenylalanine, tryptophan, threonine and histidine except valine, and optionally comprising a non-essential amino acid(s).
[2] The nutrition composition according to item [1], wherein the nutrition composition comprises at least methionine as the essential amino acid.
[3] The nutrition composition according to item [1], wherein the nutrition composition comprises no non-essential amino acid.
[4] The nutrition composition according to item [1], wherein the nutrition composition comprises at least one non-essential amino acid selected from the group consisting of arginine, glycine, serine, asparagine and glutamine.
[5] The nutrition composition according to item [1], wherein the nutrition composition further comprises a nutrient other than the amino acids.
[6] The nutrition composition according to item [1], wherein the nutrition composition is to be taken for 11 days or more.
[7] The nutrition composition according to item [1], wherein the nutrition composition is 1) selected from a solid food, a solid agent, a semi-solid food, a semi-solid agent, a beverage, and a liquid, or is 2) a culture medium.
[8] A kit comprising: the nutrition composition according to item [1]; and a cell population containing cells differentiated from stem cells.
[9] A method for suppressing formation and/or proliferation of undesired cells derived from stem cells, comprising allowing a cell population containing cells differentiated from stem cells to take the nutrition composition according to item [1].
[10] Use of the nutrition composition according to item [1] for suppressing formation and/or proliferation of undesired cells derived from stem cells in a cell population containing cells differentiated from stem cells.
[11] A method for suppressing formation and/or proliferation of undesired cells derived from stem cells *in vivo,* comprising allowing a mammal, to which a cell population containing cells differentiated from stem cells has been transplanted or administered, to take the nutrition composition according to item [1].

### Advantageous Effects of Invention

Intake of the nutrition composition of the present invention enables to suppress formation and/or proliferation of undesired cells derived from stem cells in a cell population containing cells differentiated from stem cells to be used in, e.g., cell therapy and regenerative medicine, without using a medicinal agent that may have adverse effects on cells and side effects on a living body, or a cumbersome treatment, with the result that a predetermined therapeutic effect by the desired cell transplanted or administrated can be obtained. For example, if a patient who received a transplant surgery of a cell population containing cells differentiated from stem cells, takes the nutrition composition of the present invention, it is possible to prophylactically or therapeutically suppress formation of undesired cells (formation of e.g., teratoma) and/or proliferation of undesired cells (proliferation of e.g., undifferentiated stem cells (e.g., iPS cells) and cells that failed to differentiate into desired cells (e.g., endoderm, mesoderm, ectoderm)) .

### Brief Description of Drawings

[Figure 1] Figure 1 shows the graphs separately representing (A) weight of the kidney transplanted to mice, (B) the weight change of mice after completion of transplant surgery and (C) weight of teratoma, in Experimental Example 1 (Transplantation Experiment 1) using a valine deficient feed.
[Figure 2] Figure 2 shows the graphs separately representing (A) weight of the kidney transplanted to mice, (B) the weight change of mice after completion of transplant surgery and (C) weight of teratoma, in Experimental Example 2 (Transplantation Experiment 2) using a serine/glycine deficient feed.
[Figure 3] Figure 3 shows the graphs separately representing (A) weight of the kidney transplanted to mice, (B) the weight change of mice after completion of transplant surgery and (C) weight of teratoma, in Experimental Example 2 (Transplantation Experiment 2) using a non-essential amino acid deficient feed.
[Figure 4] Figure 4 is a graph showing the survival rate of human iPS cells in each of a valine-containing medium (+valine) and a valine-free medium (-valine) in Experimental Example 4.
[Figure 5] Figure 5 is a graph showing the survival rate of human iPS cells cultured together with organoid in each of a valine-containing medium (+valine) and a valine-free medium (-valine) in Experimental Example 5.

### Description of Embodiments

In the specification, the "stem cell(s)" refers to, for example, a pluripotent stem cell(s) and a multipotent stem cell(s).

The "pluripotent stem cell(s)" refer to a stem cell(s) capable of differentiating into various tissues and a cell(s) different in form and function in a living body and having an ability to differentiate into any lineage cell(s) of three germ layers (endoderm, mesoderm, ectoderm). Examples of the "pluripotent stem cell(s)" that can be used in the present invention include, but are not particularly limited to, an embryonic stem cell(s) (an ES cell(s), sometimes referred to as "an ESC(s)" herein), an embryonic stem cell(s) derived from a cloned embryo obtained by nuclear transplantation, a sperm stem cell(s), an embryonic germ cell(s) and an induced pluripotent stem cell(s) (an iPS cell(s), sometimes referred to as "an iPSC(s)" herein).

The "induced pluripotent stem cells (iPSCs)" refer to cells obtained by introducing predetermined factors (nuclear reprogramming factors) into a mammalian somatic cell or an undifferentiated stem cell and reprogramming the cell. At present, various types of "induced pluripotent stem cells" are known. Examples of the iPSC cells that can be used include iPSC established by Yamanaka et al., by introducing four factors: Oct3/4, Sox2, Klf4 and c-Myc, into a mouse fibroblast cell (Takahashi K, Yamanaka S., Cells, (2006) 126: 663-676); human cell-derived iPSC established by introducing the same four factors into a human fibroblast cell (Takahashi K, Yamanaka S., et al. Cells, (2007) 131: 861-872); Nanog-iPS cells established by introducing the four factors, and then, screening the cells based on expression of Nanog (Okita, K., Ichisaka, T., and Yamanaka, S. (2007), Nature 448, 313-317); iPS cells prepared by a method without using c-Myc (Nakagawa M, Yamanaka S., et al. Nature Biotechnology, (2008) 26, 101-106); and iPS cells established by introducing six factors in accordance with a virus-free method (Okita K et al. Nat. Methods 2011 May; 8 (5): 409-12, Okita K et al. Stem cells, 31 (3) : 458-66). Also, induced pluripotent stem cells established by introducing four factors: Oct3/4, Sox2, NANOG and LIN28, prepared by Thomson et al., (Yu J., Thomson JA. et al., Science (2007) 318: 1917-1920); induced pluripotent stem cells produced by Daley et al., (Park IH, Daley GQ. et al., Nature (2007) 451: 141-146); and induced pluripotent stem cells produced by Sakurada et al. (JP 2008-307007 A), can be used. Other than those mentioned above, induced pluripotent stem cells described in all published papers (e.g., Shi Y., Ding S., et al., Cellstem Cells, (2008) Vol3, Issue 5, 568-574; Kim JB., Scholer HR., et al., Nature, (2008) 454, 646-650; Huangfu D., Melton, DA., et al., Nature Biotechnology, (2008) 26, No 7, 795-797) and induced pluripotent stem cells known in the art described in patents (e.g., JP2008-307007, JP2008-283972, US2008-2336610, US2009-047263, WO2007-069666, WO2008-118220, WO2008-124133, WO2008-151058, WO2009-006930, WO2009-006997, WO2009-007852), can be all used.

As the "induced pluripotent stem cells (iPSCs)", iPSC strains established by e.g., NIH, RIKEN (the Institute of Physical and Chemical Research) and Kyoto University, can be used. Examples of the human iPSC strains include strains produced by RIKEN such as HiPS-RIKEN-1A strain, HiPS-RIKEN-2A strain, HiPS-RIKEN-12A strain and Nips-B2 strain; and strains produced by Kyoto University such as 201B7 strain, 253G1 strain, 253G4 strain, 409B2 strain, 454E2 strain, 606A1 strain, 610B1 strain, 648A1 strain, 1201C1 strain, 1205D1 strain, 1210B2 strain, 1231A3 strain, 1383D2 strain and 1383D6 strain. Alternatively, clinical-grade cell strains provided by, e.g., Kyoto University and Cellular Dynamics International, and cell strains for research and clinical use prepared by these clinical-grade cell strains, may be used.

The examples of available "embryonic stem cells (ESCs)" include mouse ESC strains established by inGenious targeting laboratory and RIKEN (the Institute of Physical and Chemical Research); and human ESC strains established by NIH, RIKEN, Kyoto University and Cellartis. Examples of the human ESC strain that can be used include strains established by NIH, such as CHB-1 to CHB-12 strains, RUES1 strain, RUES2 strain, and HUES1 to HUES28 strains; strains established by WisCells Research, such as H1 strain, H9 strain; strains established by RIKEN such as KhES-1 strain, KhES-2 strain, KhES-3 strain, KhES-4 strain, KhES-5 strain, SSES1 strain, SSES2 strain, SSES3 strain. Alternatively, clinical-grade cell strains and cell strains for research and clinical use produced by the clinical-grade cell strains, may be used.

The "multipotent stem cells" refer to stem cells having an ability to differentiate into cells of a plurality of limited numbers of cell lineages. Examples of the "multipotent stem cells" that can be used in the present invention and classified based on the lineage into which the stem cells can be differentiated, include mesenchymal stem cells, hematopoietic stem cells, neural stem cells and epithelial stem cells (cultured fibroblasts). Examples of the "multipotent stem cells" that are classified based on the tissues from which the stem cells are collected (derived), include dental pulp stem cells, oral mucosa-derived stem cells, hair follicle stem cells, bone marrow stem cells, and somatic stem cells derived from the adipose tissue, umbilical blood, placenta and other tissues.

The "mesenchymal stem cells" refer to multipotent stem cells capable of differentiating into the mesenchymal cells including osteoblasts, muscle cells, chondrocytes and adipose cells. In the present invention, the mesenchymal stem cells may be cells isolated from a living tissue or cells derived from ES cells and iPS cells. Examples of the markers specific to the mesenchymal stem cells include, but at not limited to, those described, for example, in Vasileios Karantalis and Joshua M. Hare, Circ Res., 2015 April 10; 116 (8): 1413-1430, and Imran Ullah, et al., Biosci. Rep., (2015), 35/art: e00191.

The "neural stem cells" refer to multipotent stem cells capable of differentiating into central neuronal cells such as neurons and glial cells (astrocytes, oligodendrocytes). In the present invention, the neural stem cells may be cells isolated from a living tissue such as the periphery of the lateral ventricle, or cells derived from ES cells and iPS cells.

The "hematopoietic stem cells" refer to multipotent stem cells capable of differentiating into hematopoietic cells. In humans, the hematopoietic stem cells are mainly present in the bone marrow and differentiating into white blood cells (neutrophils, eosinophils, basophils, lymphocytes, monocytes, macrophages), red blood cells, platelets, mast cells and dendritic cells. In the present invention, the hematopoietic stem cells may be cells isolated from a living tissue such as the bone marrow, and derived from ES cells and iPS cells.

In the specification, the "cell population" refers to two or more cells of the same type or different types. The "cell population" also refers to a mass formed of the same type of cell or different types of cells. Examples of the "cell population" include an organ bud of an organ formed of a plurality of types of cells. As to such an organ bud, see WO2013/047639 (organ buds of the liver and pancreatic β cells), WO2015/012158 (organ buds of the liver, pancreatic β cells, kidney, intestine and lung).

The term "comprise(s) or comprising" means that elements following this term are included; but the elements to be included are not limited to those described. In other words, inclusion of the elements following the term is suggested but exclusion of other arbitrary elements is not suggested.

In the specification, the terms "deplete" and "depletion" mean that the amount of a predetermined component in a composition such as a cellular composition decreases. The term "depleted" when it is used for explaining, a cellular composition such as a cell population, means that the amount of a predetermined component decreases compared to the ratio of the component in the cell population before depletion. For example, in a composition such as a cell population, a target cell type (herein, undesired cells derived from stem cells of the present invention, in particular, undifferentiated stem cells) can be depleted. Accordingly, the ratio of target cell type decreases compared to the ratio of the target cell type in a cell population before depletion. In a cell population, target cell type can be depleted by a selection or screening method known in the technical field. A cell population can be depleted by a predetermined screening or selection process described in the specification. In a predetermined embodiment of the present invention, a target cell population is reduced (depleted) up to at least 50%, 80%, 85%, 90%, 95%, 97%, 98%, 99% or 99.9% relative to a cell population by a method for depleting the target cell population.

In the specification, "purify" and "purification" refer to rendering a predetermined component pure by removing impurities in a composition such as a cellular composition. The "purified", when it is used for explaining a cellular composition such as a cell population, means that the amount of impurities reduces compared to the ratio of the impurities in the cell population before purification, and the purity of a predetermined component improves. For example, in a composition such as a cell population, target cell type (herein, desired cells differentiation-induced from the stem cells of the present invention) can be purified. Accordingly, the ratio of target cell type increases compared to the ratio of the target cell type in the cell population before purification. The target cell type in a cell population can be purified by a selection or screening method known in the technical field. A cell population can be purified by a predetermined screening or selection process described in the specification. In a predetermined embodiment of the present invention, the purity of a target cell population may be increased up to at least 70%, 80%, 85%, 90%, 95%, 97%, 98%, 99% or 99.9% or conversely to say, the ratio of impurities (cells serving as a contaminant) can be reduced up to detection limit or less, by a method of purifying a target cell population.

In the specification, "culture" means that cells are kept in an *in-vitro* environment, proliferated (grown) and/or differentiated. "To culture" means that cells are maintained outside a tissue or a living body, for example, in a cell-culture dish or a flask, proliferated (grown) and/or differentiated.

Examples of the culture medium that is generally used in the present invention include BME culture medium, BGJb culture medium, CMRL1066 culture medium, Glasgow MEM culture medium, Improved MEM (IMEM) culture medium, Improved MDM (IMDM) culture medium, Medium 199 culture medium, Eagle MEM culture medium, αMEM culture medium, DMEM culture medium (high glucose, low glucose), DMEM/F12 culture medium, Ham culture medium, RPMI 1640 culture medium, Fischer's culture medium, AK02N culture medium, E8 supplement culture medium, Stempro-34 SFM, HCM and mixed culture medium of these.

Examples of a culture medium for ES cells and iPS cells include DMEM containing 10 to 15% FBS, DMEM/F12 or DME culture solution (these culture solutions may further appropriately contain, e.g., LIF, penicillin/streptomycin, puromycin, L-glutamine, non-essential amino acids and β-mercaptoethanol) and a culture solution for commercially available iPS cells such as a cell culture solution for mouse ES cells (TX-WES culture solution, THROMBO X), a cell culture solution for primates ES cells (cell culture solution for primates ES/iPS cells, REPROCELL), a serum-free medium (mTESR, Stemcell Technology) and iPS/ES medium for cell proliferation/regenerative medicine (StemFit (registered trademark), Ajinomoto Healthy Supply Co., Inc). Other than these, a serum-free culture medium may be used for culture (Sun N, et al. (2009), Proc Natl Acad Sci USA. 106: 15720-15725).

A method for culturing mouse iPS cells is described in Takahashi K, Yamanaka S., Cells, (2006) 126: 663-676 and Takahashi K, Okita K, et al. Nat Protoc. 2007; 2 (12): 3081-9. A method for culturing human iPS cells on SNL feeder cells is described in Takahashi K, Yamanaka S., et al. Cells, (2007) 131: 861-872, and Ohnuki M, Takahashi K, Yamanaka S. Curr Protoc Stem Cells Biol. (2009). A method for culturing human iPS cells on MEF feeder is described in Yu J., Thomson JA. et al., Science (2007) 318: 1917-1920. A method for culturing human iPS cells by using autologous fibroblasts as a feeder is described in Takahashi K, et al. PLoS One 4, e8067 (2009). A method for culturing human ES/iPS cells in a feeder-free medium is described in Rodin S et al., Nat Biotechnol. (2010) 28 (6): 611-5, Chen et al., Nat Methods (2011) 8 (5): 424-429, Miyazaki, T. et al. Nat Commun (2012) 3, 1236, Okita et al., Stem cells, (2013) 31 (3): 458-66, and Nakagawa M et al., Scientific Reports, (2014) 4: 3594. A method for culturing human ES/iPS cells in a large scale is described in Olmer R, et al., Tissue Eng Part C Methods. 2012 Oct; 18 (10): 772-84, Wang Y et al., Stem Cells Res. 2013 Nov; 11 (3): 1103-16, and Otsuji T, et al., Stem Cells Reports. 2014 Apr 24; 2 (5): 734-45. A method for culturing human ES cells is described in Thomson, J. A. et al. Science (1998) 282, 1145-1147, and Amit M. et al. Dev Biol. 2000 Nov 15; 227 (2): 271-8. A method for culturing human ES cells in a feeder free medium is described in Xu, C. et al., Nat Biotechnol (2001) 19, 971-974.

In an embodiment of the present invention, cell culture may be carried out by adhesive culture without using feeder cells. For culturing, a culture vessel, such as a dish, a flask, a microplate and a cell-culture sheet including OptiCells (product name) (Nunc), is used. It is preferable that a surface treatment for improving adhesiveness (hydrophilicity) to cells is applied to a culture vessel or that a culture vessel is coated with a cell-adhesive substrate such as collagen, gelatin, poly-L-lysine, poly-D-lysine, laminin, fibronectin, Matrigel (examples: BD Matrigel (Becton, Dickinson and Company)) and vitronectin.

In an embodiment of the present invention, cells may be cultured by suspension culture. The suspension culture is carried out in a culture solution while stirring or shaking it to homogenize culture-solution components and oxygen concentration therein, and cells are proliferated through formation of aggregates. The stirring rate is appropriately set suitably depending on the cell density and the culture-vessel size. Excessive stirring or shaking gives physical stress to cells and inhibits formation of cell aggregates. Thus, stirring or shaking rate is controlled in such a manner that the culture-solution components and oxygen concentration in a culture solution can be homogenized and formation of aggregates is not inhibited.

The culture temperature, which is not particularly limited, is generally 30 to 40°C (e.g., 37°C). The carbon-dioxide concentration in a culture vessel, which is not particularly limited, is, for example, about 5%; and the oxygen concentration (not particularly limited) is generally about 1% to 21%.

The "growth factor" refers to an endogenous protein promoting differentiation and/or proliferation of a predetermined cell. Examples of the "growth factor" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transforming growth factor beta (TGF-β), vascular endothelial growth factor (VEGF), transferrin, various interleukins (e.g., IL-1 to IL-18), various colony-stimulating factors (e.g., granulocytes/macrophage colony-stimulating factor (GM-CSF)), various interferons (e.g., IFN-y) and other cytokines having an effect on stem cells such as stem cells factor (SCF) and erythropoietin (Epo).

In the specification, *"ex vivo"* is used for expressing that experiments or measurements were carried out in a living tissue such as a cultured tissue or cultured cells placed in an artificial environment outside a living body. The tissue or cells to be used may be frozen for preservation and thawed later for use in an *ex-vivo* treatment. If a tissue-culture experiment of living cells or tissue is carried out continuously for several days or more, the term "*in vitro"* is used. The term "*in vitro"* is sometimes interchangeably used with *"ex-vivo".* In contrast, the term *"in vivo"* is generally used for referring to a phenomenon such as proliferation of cells that occurs within a living body.

In the specification, unless otherwise specified, the term "essential amino acids" refers to essential amino acids for humans (adult) (human essential amino acids), and more specifically, 9 types of amino acids, i.e., valine (V), isoleucine (I), leucine (L), methionine (M), lysine (K), phenylalanine (F), tryptophan (W), threonine (T) and histidine (H). Of them, a group consisting of "isoleucine, leucine, methionine, lysine, phenylalanine, tryptophan, threonine and histidine" except valine, is referred to as a "specified essential amino acid group" in the specification.

In the specification, unless otherwise specified, the term "non-essential amino acids" refers to non-essential amino acids for humans (adult) (human non-essential amino acids), and more specifically, 11 types of amino acids, i.e., arginine (R), glycine (G), serine (S), asparagine (N), glutamine (Q), alanine (A), cysteine (C), aspartic acid (D), glutamic acid (E), tyrosine (Y) and proline (P). Of them, a group consisting of "arginine, glycine, serine, asparagine and glutamine" is referred to as a "specified non-essential amino acid group" in the specification.

Note that, unless otherwise specified, essential amino acids and non-essential amino acids have L-form available for humans and other mammals, and may be in the form of a salt and/or a derivative (e.g., L-histidine hydrochloride, L-lysine hydrochloride, N-acetyl-L-cysteine, L-cystine, N-acetyl-L-tryptophan).

### (Nutrition composition)

The nutrition composition of the present invention is a nutrition composition for suppressing formation and/or proliferation of undesired cells derived from stem cells in a cell population containing cells differentiated from stem cells, the nutrition composition comprising at least one essential amino acid selected from the group (specified essential amino acid group) consisting of isoleucine, leucine, methionine, lysine, phenylalanine, tryptophan, threonine and histidine except valine, and optionally comprising a non-essential amino acid(s).

### (Amino acid component)

The phrase "comprising at least one essential amino acid selected from the group consisting of isoleucine, leucine, methionine, lysine, phenylalanine, tryptophan, threonine and histidine except valine" means that valine of the essential amino acids is not contained, and at least one (may be single, several or all) selected from the group consisting of "isoleucine, leucine, methionine, lysine, phenylalanine, tryptophan, threonine and histidine" (specified essential amino acid group) is contained and amino acids not selected are not contained.

Accordingly, in an embodiment, the nutrition composition of the present invention does not contain valine (deficient in valine) and contains all essential amino acids belonging to the specified essential amino acid group; in other words, the nutrition composition is a composition in which only valine of the essential amino acids is not contained (deficient in valine).

The nutrition composition of the present invention may be a composition containing methionine at least as an essential amino acid. More specifically, methionine of the specified essential amino acid group is at least selected as the component to be contained in a nutrition composition; the other essential amino acids may be selected (contained in the nutrition composition of the present invention) or not selected (not contained in the nutrition composition of the present invention) as the amino acids to be contained in the nutrition composition.

Accordingly, in an embodiment, the nutrition composition of the present invention may be a nutrition composition which does not contain valine (deficient in valine) and contains at least methionine of the specified essential amino acid group, and may or may not contain the other amino acids (may or may not select them as an essential amino acid).

The nutrition composition of the present invention optionally contains a non-essential amino acid(s); in other words, at least one of amino acids corresponding to the non-essential amino acids may or may not be contained. Note that, the essential amino acids to be contained in the nutrition composition of the present invention may refer to the description of the specification.

In an embodiment, the nutrition composition of the present invention does not contain all non-essential amino acids. In the embodiment, the essential amino acids except valine may be all contained (all essential amino acids belonging to the specified essential amino acid group are contained) or essential amino acids may be contained in accordance with the descriptions of the above two embodiments described in connection with essential amino acids.

In an embodiment, the nutrition composition of the present invention may be a nutrition composition containing at least one non-essential amino acid selected from the group consisting of "arginine, glycine, serine, asparagine and glutamine" (specified non-essential amino acid group); and more specifically, at least one (may be single, several or all) selected from the specified non-essential amino acid group may be contained and the non-essential amino acids not selected are not contained. The nutrition composition of the present invention contains, for example, (i) arginine, (ii) glycine and serine, (iii) arginine and glutamine, (iv) asparagine and glutamine or (v) arginine, glycine, serine, asparagine and glutamine of the specified non-essential amino acid group and does not contain specified non-essential amino acids except the above (i) to (v), and, if necessary, may contain a non-essential amino acid not belonging to the specified non-essential amino acid group (at least one selected from the group consisting of alanine, cysteine, aspartic acid, glutamic acid, tyrosine and proline (P)).

The suppressing "formation and/or proliferation of undesired cells derived from stem cells" may be carried out *in vivo, ex-vivo* or *in vitro.* Suppressing formation and/or proliferation of undesired cells derived from stem cells *in vivo* is, for example, suppressing formation and/or proliferation of undesired cells derived from stem cells in a mammal such as a human, to which a cell population containing cells differentiated from stem cells is transplanted or administered. Suppressing formation and/or proliferation of undesired cells derived from stem cells *ex-vivo* is, for example, suppressing formation and/or proliferation of undesired cells derived from stem cells in a culturing stage of a cell population to be used for the aforementioned transplantation. Accordingly, the nutrition composition of the present invention can take a form of a meal or diet suitable for feeding (administering, eating) the composition to a mammal such as a human, in preparation for the former case where suppression is carried out *in vivo,* or can take a form for culture, which is suitable for cells to take (absorb the form from a culture medium), in preparation for the latter case where suppression is carried out *ex vivo.*

Now, the "cell population containing cells differentiated from stem cells", which is a target of suppressing "formation and/or proliferation of undesired cells derived from stem cells" will be described. Then, typical embodiments of the nutrition composition of the present invention, that is, an embodiment suitable for allowing a mammal such as a human to take the composition so as to produce the functional effect of the present invention *in vivo* (the nutrition composition of the embodiment will be referred to as "first nutrition composition" herein), and an embodiment suitable for culturing cells so as to produce the functional effect of the present invention *ex vivo* (the nutrition composition of the embodiment will be referred to as "second nutrition composition" herein), will be described sequentially in the order.

### (Cell population containing cells differentiated from stem cells)

The "cell population containing cells differentiated from stem cells" is typically a cell population that is produced in the middle or final stage of a culture process for differentiation induction of the stem cells into desired cells and that is a mixture of desired cells differentiation-induced from the stem cells and undesired cells (e.g., undifferentiated stem cells (e.g., iPS cells)) which fail to differentiate into desired cells and cells (e.g., endoderm, mesoderm, ectoderm) stopped differentiation into desired cells in the middle of a culture process.

In embodiments of the first and second nutrition composition, the "cell population containing cells differentiated from stem cells" is intended to be used in transplanting or administering to a mammal for the purpose of cell therapy or regenerative medicine.

Note that, a method for using the "cell population containing cells differentiated from stem cells", for example, a method for transplanting or administering the cell population to a target mammal; and other technical matters involved in such a cell population may be referred to routine methods, and various embodiments known in the art can be used.

In another embodiment of the second nutrition composition, the "cell population containing cells differentiated from stem cells" is not limited to a cell population prepared for therapeutic use such as cell therapy or regenerative medicine, and may be prepared for other uses (e.g., constructing a drug screening system and a toxicity evaluation system).

The "cell population containing cells differentiated from stem cells" in a cell population includes desired cells and undesired cells, which can be arbitrarily selected from various types of cells depending on the use of the cell population and culture method thereof.

Examples of the "desired cells" include splenic cells, nerve cells, glial cells, pancreatic β cells, bone marrow cells, mesangium cells, Langerhans cells, epidermal cells, epithelial cells, endothelial cells, fibroblasts, muscle cells (examples: skeletal muscle cells, cardiomyocytes, myoblasts, muscle satellite cells), fat cells, immune cells (examples: macrophages, T cells, B cells, natural killer cells, mast cells, neutrophils, basophils, eosinophils, monocytes, megakaryocytes), synovial cells, chondrocytes, bone cells, osteoblasts, osteoclasts, mammary gland cells, hepatocytes, stromal cells, egg cells and sperm cells, may be matured cells differentiated or functional progenitor cells.

Typical examples of the desired cells include cells for use in regenerative medicine using iPS cells, such as dopamine-producing cells, neural stem cells, cornea, retinal pigment epithelial cells, myocardium, photoreceptor cells, platelet, red blood cells, bone, cartilage, skeletal muscle, kidney cells, pancreatic β cells, hepatocytes and functional progenitor cells of these. In the "functional progenitor cells" refer to cells having the same or analogous effect as the corresponding mature cells.

In contrast, examples of the "undesired cells" include cells exerting no intended (expected) effect and/or having a possibility of exerting an undesirable effect compared to the desired cells, such as undifferentiated or immature cells (e.g., undifferentiated stem cells (e.g., iPS cells), cells stopped differentiation into desired cells in the middle of a culture process) (e.g., endoderm, mesoderm, ectoderm), and cells unintentionally differentiated (e.g., teratoma). The "undesired cells" also include undifferentiated stem cells which fail to differentiate during a differentiation induction process from the stem cells into desired cells.

In the present invention, a cell population containing cells differentiated from stem cells (e.g., a cell population to be transplanted or administered for cell therapy and regenerative medicine) is preferably a cell population prepared by depleting undifferentiated stem cells (e.g., iPS cells) and other undesired cells (e.g., cells (e.g., endoderm, mesoderm, ectoderm) stopped differentiation into desired cells in the middle of a culture process) as much as possible (content of these cells is reduced); conversely to say, a cell population containing desired cells in a high purity (content or purity of desired cells is increased as much as possible). The level of such "depletion" or "purification" is as separately described in the specification.

In an embodiment of the first nutrition composition, the content of undesired cells derived from stem cells in a cell population falls within a preferable range of suppressing formation of undesired cells (e.g., formation of teratoma) and/or suppressing proliferation of undesired cells (proliferation of e.g., undifferentiated stem cells (e.g., iPS cells) and cells stopped differentiation into desired cells (e.g., endoderm, mesoderm, ectoderm) in the middle of a culture process) by intake of the first nutrition composition described later. More specifically, the content of undesired cells in a cell population is appropriately determined by those skilled in the art depending on the desired therapeutic effect, and is not generally defined.

In an embodiment of the second nutrition composition, formation and/or proliferation of undesired cells derived from stem cells (e.g., undifferentiated stem cells (e.g., iPS cells) and cells (e.g., endoderm, mesoderm, ectoderm) stopped differentiation into desired cells in the middle of a culture process) in a cell population containing cells differentiated from stem cells, can be suppressed *in vitro* by using the second nutrition composition described later. In the embodiment, another depletion (purification) means can be used in combination with the second nutrition composition. Examples of the depletion (purification) means to be used in combination, include differentiation induction conditions (e.g., temperature, oxygen concentration, carbon dioxide concentration), culture medium components except amino acids and culture methods. By adjusting (optimizing) these means, differentiation induction efficiency from stem cells into desired cells may be enhanced, thereby reducing the content of undesired cells such as remaining stem cells. Furthermore, if necessary, a depletion (purification) means for collecting differentiated cells (cells expressing a marker specific to differentiated cells) by sorting using flow cytometry and a depletion (purification) means for removing stem cells in a cell population by expression of a suicide gene in a chemical-agent induction manner, may be used.

In the embodiment of the second nutrition composition, differentiation induction conditions, culture medium components, culture method and other technical matters for preparing a cell population containing predetermined desired cells from stem cells except use of the second nutrition composition described later may refer to routine methods. Various embodiments known in the art can be used.

### (First nutrition composition)

The form of a first nutrition composition, which is to be fed to a mammal such as a human, is not particularly limited as long as it is a form that can be orally or parenterally administered. Examples of the form of the composition may be a solid food, a solid agent (e.g., solid or powder for oral ingestion), a semi-solid food (e.g., jelly, fluid food), a semi-solid agent (e.g., jelly for oral intake), a beverage and a liquid (e.g., liquid for oral ingestion or liquid for parenteral ingestion (e.g., infusion preparation)).

The content of amino acids in a first nutrition composition, which can be appropriately adjusted depending on the types of amino acids, is for example, 1.25 to 12.5 g/100 kcal nutrition composition per energy of the whole nutrition composition.

The contents of essential amino acids (other than the amino acid to be purposely depleted) in a first nutrition composition can be appropriately adjusted by referring to daily intake per adult per day, recommended by WHO (FAO/WHO/UNU (2007), "PROTEIN AND AMINO ACID REQUIREMENTS IN HUMAN NUTRITION", WHO Press, p.150) shown in the following Table in consideration of intake form of the nutrition composition of the present invention (e.g., intake of a nutrition composition and amount of energy per day or per time). Note that, the intake for a child of 3 years old or more is 10 to 20% as high as that for an adult and the intake for a baby less than one year old is 150% as high as that for an adult. Cysteine and tyrosine are not essential amino acids and classified in non-essential amino acids; however, since the recommended intakes of cysteine and tyrosine are defined as the total intakes including the recommended intakes of methionine and phenylalanine, respectively, the total intakes are listed in the table. The contents of cysteine and tyrosine can be adjusted in consideration of the intake form of the nutrition composition of the present invention, (e.g., intake of a nutrition composition, amount of energy per day or per time) and with reference to the above Table.

**[Table 1]**

| Essential amino acid | Recommended intake per day for an adult |
|---|---|
| Valine | 26 mg/kg of body weight |
| Isoleucine | 20 mg/kg of body weight |
| Leucine | 39 mg/kg of body weight |
| Lysine | 30 mg/kg of body weight |
| Methionine+ cysteine | Total 15 mg/kg of body weight |
| | (10.4+4.1) |
| Phenylalanine + tyrosine | Total 25 mg/kg of body weight |
| Tryptophane | 4 mg/kg of body weight |
| Threonine | 15 mg/kg of body weight |
| Histidine | 10 mg/kg of body weight |

The content of non-essential amino acids except cysteine and tyrosine can be appropriately adjusted in consideration of intake form (e.g., intake of a nutrition composition, amount of energy per day or per time) of the nutrition composition of the present invention.

The contents of individual essential amino acids in a first nutrition composition when the composition is given to humans, are, for example, as follows:
Valine: 0 mg/kg body weight;
Isoleucine: 0 mg/kg body weight to 30 mg/kg body weight, preferably, 0 mg/kg body weight to 20 mg/kg body weight;
Leucine: 0 mg/kg body weight to 50 mg/kg body weight, preferably, 0 mg/kg body weight to 40 mg/kg body weight;
Methionine: 0 mg/kg body weight to 30 mg/kg body weight, preferably, 0 mg/kg body weight to 15 mg/kg body weight;
Lysine: 0 mg/kg body weight to 50 mg/kg body weight, preferably, 0 mg/kg body weight to 30 mg/kg body weight
Phenylalanine: 0 mg/kg body weight to 50 mg/kg body weight, preferably, 0 mg/kg body weight to 25 mg/kg body weight;
Tryptophan: 0 mg/kg body weight to 30 mg/kg body weight, preferably, 0 mg/kg body weight to 5 mg/kg body weight;
Threonine: 0 mg/kg body weight to 30 mg/kg body weight, preferably, 0 mg/kg body weight to 15 mg/kg body weight;
Histidine: 0 mg/kg body weight to 30 mg/kg body weight, preferably, 0 mg/kg body weight to 10 mg/kg body weight.

In producing a first nutrition composition, a production comprising determining the content ratio of essential amino acids and non-essential amino acids to be contained in the nutrition composition, for example, as mentioned above; blending commercially available individual amino acid materials to prepare an amino acid mixture; and forming the mixture into a composition having a desired form, is preferably employed in view of handleability. By further blending the amino acid mixture mentioned above with other optional components described later, a first nutrition composition can be efficiently produced.

### (Other components)

A first nutrition composition may further contain nutrients (other components) other than amino acids. In consideration that a mammal, to which a cell population containing cells differentiated from stem cells is transplanted or administered, thereafter continuously takes a first nutrition composition for a certain period, it is preferable that the first nutrition composition further contains other types of nutrients in addition to the predetermined amino acids. In other words, it is preferable that all nutrients necessary for the mammal can be taken from the first nutrition composition alone.

Examples of the nutrients other than amino acids that a first nutrition composition can contain, include fats and oils, sugar, minerals (inorganic salts, trace elements) and vitamins.

A fat and oil (lipid) are mainly constituted of a compound (fatty acid triglyceride) formed of glycerol and fatty acid via an ester bond. The fatty acids, which are components of lipids, can be classified into saturated fatty acids (fatty acids having no double bond in a molecule), monounsaturated fatty acids (fatty acids having a single double bond in a molecule) and polyunsaturated fatty acids (fatty acids having two or more double bonds in a molecule). Of the polyunsaturated fatty acids, linoleic acid and α-linolenic acid, which are not synthesized inside an animal body and must be taken from foods, are referred to as essential fatty acids and preferably contained in a first nutrition composition.

Specific examples of the saturated fatty acids, monounsaturated fatty acids and polyunsaturated fatty acids include the following compounds (the numbers within parentheses represent "the number of carbon atoms: the number of double bonds"; "n-3", "n-6", "n-7"and "n-9" represent the 3rd, 6th, 7th and 9th positions of the carbon atom, which are counted from the carbon atom of the methyl group at an end; at which a double bond first appears, and sometimes represent ω3, ω6, ω7 and ω9, respectively): butanoic acid (butyric acid, 4: 0), hexanoic acid (caproic acid, 6: 0), heptanoic acid (7: 0), octanoic acid (caprylic acid, 8: 0), decanoic acid (capric acid, 10: 0), dodecanoic acid (lauric acid, 12: 0), tridecanoic acid (13: 0), tetradecanoic acid (myristic acid, 14: 0), pentadecanoic acid (15: 0), hexadecane acid (palmitic acid, 16: 0), heptadecane acid (17: 0), octadecane acid (stearic acid 18: 0), icosanoic acid (arachidic acid, 20: 0), docosanoic acid (behenic acid, 22: 0), tetraicosanoic acid (lignoceric acid, 24: 0), decenoic acid (10: 1), tetradecenoic acid (myristoleic acid, 14: 1), pentadecenoic acid (15: 1), hexadecenoic acid (palmitoleic acid, 16: 1), heptadecenoic acid (17: 1), octadecenoic acid (oleic acid, 18: 1, n-9), octadecenoic acid (cis-vaccenic acid, 18: 1, n-7), icosenoic acid (eicosenoic acid, 20: 1), docosenoic acid (22: 1), tetracosenoic acid (24: 1), hexadecadienoic acid (16: 2), hexadecatrienoic acid (16: 3), hexadecatetraenoic acid (16: 4), heptadecadienoic acid (17: 2), octadecadienoic acid (18: 2), octadecadienoic acid (linoleic acid, 18: 2, n-6), octadecatrienoic acid (18: 3), octadecatrienoic acid (α-linolenic acid, 18: 3, n-3), octadecatrienoic acid (γ-linolenic acid, 18: 3, n-6), octadecatetraenoic acid (18: 4, n-3), icosadienoic acid (eicosadienoic acid, 20: 2, n-6), icosatrienoic acid (eicotrienoic acid, 20: 3, n-6), icosatetraenoic acid (eicosatetraenoic acid, 20: 4, n-3), icosatetraenoic acid (arachidonic acid, 20: 4, n-6), icosapentaenoic acid (eicosapentaenoic acid, 20: 5, n-3), henicosapentaenoic acid (21: 5, n-3), docosadienoic acid (22: 2), docosatetraenoic acid (22: 4, n-6), docosapentaenoic acid (22: 5, n-3), docosapentaenoic acid (22: 5, n-6) and docosahexaenoic acid (22: 6, n-3).

Examples of the fats and oils serving as supply sources for fatty acids include natural fats and oils such as soybean oil, corn oil, palm oil, perilla oil, canola oil, safflower oil, sunflower, sesame oil, rice oil, graph seed oil and fish oil; and synthetic fats and oils such as a medium chain fatty acid triglyceride (MCT) having about 6 to 12 carbon atoms. Examples of MCT include caproic acid triglyceride, dicaprylic acid/capric acid triglyceride, lauric acid/capric acid/caprylic acid triglyceride and caprylic acid triglyceride (Tricaprylin).

The content of a fat and oil in a first nutrition composition, which can be appropriately adjusted depending on the type of fat and oil, is for example, 0.1 to 5 g/100 kcal nutrition composition per energy of the whole nutrition composition.

Examples of the sugar include starch (example: cornstarch), dextrin, maltodextrin, fructo-oligosaccharide, galacto-oligosaccharide, lactosucrose, lactulose, inulin, maltose, sucrose and glucose.

The content of a sugar in a first nutrition composition, which can be appropriately adjusted depending on the type of sugar, is for example, 0.1 to 5.0 g/100 kcal per energy of the whole nutrition composition.

Examples of the mineral include, calcium, magnesium, sodium, potassium, phosphorus, iron, manganese, copper, iodine, zinc, selenium, chromium, and molybdenum, sulfur, chlorine and cobalt. These minerals may be present in the form of salt (e.g., a hydrogencarbonate (bicarbonate) such as sodium hydrogen carbonate). A preparation obtained by blending one or two or more minerals in advance (a premix) may be used, or, if necessary, further one or two or more minerals may be added to the premix and put in use.

The content of minerals in a first nutrition composition, which can be appropriately adjusted depending on the type of mineral, is for example, 1 mg to 50 g/100 kcal nutrition composition per energy of the whole nutrition composition.

Examples of vitamins include fat-soluble vitamins such as vitamin A, vitamin D, vitamin E and vitamin K; and water-soluble vitamins such as vitamin B and vitamin C. A preparation obtained by blending one or two types or more vitamins in advance (a premix) may be used, or, if necessary, further one or two or more vitamins may be added to the premix and put in use.

Examples of vitamin A include retinol (vitamin A₁), 3-dehydroretinol (vitamin A₂), retinal, 3-dehydroretinal, retinoic acid and 3-dehydroretinoic acid, derivatives of these such as acetic acid esters and palmitic acid esters of these, and provitamin A such as β-carotene. Examples of vitamin D include ergocalciferol (vitamin D₂), cholecalciferol (vitamin D₃), and derivatives of these such as sulfuric acid esters of these. Examples of vitamin E include α-tocopherol, β-tocopherol, γ-tocopherol, δ-tocopherol, α-tocotrienol, β-tocotrienol, γ-tocotrienol, δ-tocotrienol, derivatives of these such as acetic acid ester, nicotinic acid ester, phosphate esters of these and salts thereof such as α-tocopherol disodium. Examples of vitamin K include phytonadione (vitamin K₁), menaquinone (vitamin K₂) and menadione (vitamin K₃) and salts of these. Examples of vitamin B include thiamine (vitamin B₁), riboflavin (vitamin B₂), nicotinic acid, nicotinamide (all up to here are niacin; vitamin B₃), pantothenic acid (vitamin B₅), pyridoxine, pyridoxal, pyridoxamine (all up to here are vitamin B₆), biotin (vitamin B₇), folic acid (vitamin Bg), cyanocobalamin, adenosylcobalamin, methylcobalamin, sulfitocobalamin, hydroxocobalamin (all up to here are vitamin B₁₂) and salts of these. Also, choline and salts of choline (e.g., choline bitartrate, choline hydrochloride) can be included in vitamin B.

The content of vitamins in a first nutrition composition can be appropriately adjusted depending on the type of vitamin. The content of all vitamins (the weights of salts and derivative of vitamins) is, for example, 0.005 mg to 1000 mg/100 kcal nutrition composition per energy of the whole nutrition composition.

A first nutrition composition may further contain, other than the aforementioned components, an excipient, an emulsifier, a stabilizer, a pH regulator, a gelling agent, a fragrance, a coloring agent and other additives as long as they are generally contained in foods and preparations.

Examples of a mammal, to which a first nutrition composition is to be administered, include humans and mammals except humans (non-human mammals such as a mouse, a rat, a hamster, a guinea pig, a rabbit, a dog, a cat, a pig, a cow, a horse, a sheep and a monkey).

The route of administration for a first nutrition composition is not particularly limited. The administration may be oral administration (e.g., eating) and parenteral administration (e.g., intravenous administration and enteral administration using, e.g., a PEG tube). The frequency of administration may be once a day to several times a day. A first nutrition composition may be administered in an appropriate dose per time.

The dose per day of a first nutrition composition is not particularly limited as long as the intake of amino acids per day is satisfied. A first nutrition composition can be administered to an adult in a dose (in terms of the amino acid composition) of 0.001 g to 1.5 g/body weight kg/day, preferably, 0.1 g to 1.0 g/body weight kg/day. The dose can be appropriately changed up and down depending on the age, body weight and sex of an administration target (human or a mammal except a human); and the form and/or method of transplantation or administration of a cell population containing cells differentiated from stem cells.

A first nutrition composition may be administered in any period from the day when a cell population containing cells differentiated from stem cells was transplanted or administrated (refers to as "operation date" herein) as long as the period is required for suppressing formation and/or proliferation of undesired cells derived from stem cells in a cell population containing cells differentiated from stem cells.

For example, the administration period of a first nutrition composition is consecutive 7 days, 8 days, 9 days, 10 days, 11 days, 12 days, 13 days, 14 days, 21 days, 28 days, 30 days, 60 days, 90 days and 120 days from the following day (day 1) of the operation date (day 0). In an embodiment, the administration period of a first nutrition composition is 11 days or more.

The administration period can be appropriately determined based on e.g., the age, body weight, sex and symptoms of an administration target (human or a mammal except a human). The administration period is determined based on the body weight is as follows. If the body weight of an administration target is 25 g (e.g., adult male mouse), the administration period is representatively 7 days to 21 days, 7 days to 28 days, 11 days to 21 days, or 11 days to 28 days. If the body weight of an administration target is 60 kg (e.g., human adult male), the administration period is generally 90 days to 120 days. If the nutrition composition of the present invention is taken for a predetermined period, a risk of the formation and/or proliferation of undesired cells derived from stem cells is reduced even after completion of intake.

The suppressive effect on formation and/or proliferation of undesired cells derived from stem cells *in vivo* in a cell population containing cells differentiated from stem cells, for example, in the case where a first nutrition composition is taken in a mammal, can be confirmed based on reduction in weight or size of undesired cells in a cell population transplanted or administered, compared to that in the case where the first nutrition composition is not taken. Desired cells and undesired cells can be detected by use of, for example, surface markers, antigens or sugar chains specific to respective cells.

### (Second nutrition composition)

A second nutrition composition for use in culturing a cell population containing cells differentiated from stem cells representatively takes a form of a culture medium. More specifically, the form of the second nutrition composition can be determined in accordance with the form of a culture medium generally used in cell culture (particularly culture for stem cells to be differentiation-induced into desired cells) or another culture medium known in the art.

The culture medium generally contains components such as inorganic salts, a carbohydrate(s), an amino acid(s), a vitamin(s), a fatty acid(s) or a lipid(s), a protein(s) or a peptide(s), serum or its alternative and trace elements. It is particularly important to add components such as vitamins (e.g., vitamin B12, vitamin A, vitamin E, riboflavin, thiamine, biotin), fatty acids/lipids (e.g., cholesterol and other steroids), proteins/peptides (e.g., albumin, transferrin, fibronectin and fetuin) to a culture medium when a serum-free medium is employed (these components are usually supplied by the serum). A second nutrition composition contains growth factors and other components required for differentiation-inducing stem cells into desired cells. The culture medium may further contain, if necessary, an antibiotics substance (e.g., antibiotic-antimycotic, penicillin, streptomycin or a mixture of these), an antibacterial agent (e.g., amphotericin B), an antioxidant, pyruvic acid and a buffer. The formulation (types and amounts of components) of a second nutrition composition can be adjusted based on a general culture medium (particularly a culture medium for differentiation-inducing stem cells to desired cells) while adjusting amino acids in accordance with the present invention as described in the specification. Other components may be in the same manner as in a general culture medium or, if necessary, may be adjusted in correspondence to the adjustment of amino acids.

Note that, components, such as fatty acids or lipids, carbohydrates, inorganic salts, trace elements and vitamins, can be selected or adjusted (modified) so as to be adopted to a cell culture, appropriately with reference to the descriptions of the components such as fats and oils, sugar, minerals and vitamins contained in the first embodiment, in the specification.

The suppressive effect *ex vivo* on formation and/or proliferation of undesired cells derived from stem cells in a cell population containing cells differentiated from stem cells can be confirmed based on a decrease of the content (ratio) of undesired cells in the cell population or an increase of the content (ratio) of desired cells in the cell population cultured in a culture medium of second nutrition composition, compared to a control case where the cell population is not cultured in a culture medium of the second nutrition composition (cultured in a control medium). The desired cells and undesired cells can be distinguished by use of, for example, a surface marker, an antigen or a sugar chain specific to each of the cell types.

### (Kit)

A kit according to the present invention contains the nutrition composition of the present invention and a cell population containing cells differentiated from stem cells. In the specification, technical matters described in connection with the nutrition composition of the present invention can be also applied to the case in connection with the kit of the present invention using the nutrition composition. For example, the kit of the present invention may contain a cell population to be used in transplant surgery for cell therapy or regenerative medicine and a first nutrition composition, which is to be taken by the human (patient) or a mammal except a human (experimental animal) who received the surgery, after the transplant surgery.

A method for suppressing formation and/or proliferation of undesired cells derived from stem cells according to the present invention comprises allowing a cell population containing cells differentiated from stem cells to take the nutrition composition of the present invention. In the specification, technical matters described in connection with the nutrition composition of the present invention can be also applied to the case in connection with the method using the nutrition composition. For example, the method for suppressing formation and/or proliferation of undesired cells derived from stem cells according to the present invention may be carried out *in vivo* or *ex vivo.*

Use of the nutrition composition according to the present invention is the use of the nutrition composition of the present invention for suppressing formation and/or proliferation of undesired cells derived from stem cells in a cell population containing cells differentiated from stem cells. In the specification, technical matters described in connection with the nutrition composition of the present invention can be also applied to the case in connection with use of the nutrition composition. For example, use of the nutrition composition according to the present invention may be carried out *in vivo* or *ex vivo.*

In the following Examples, the present invention will be further specifically described by way of Experimental Examples (Transplantation Examples); however, the present invention is not limited by these Examples.

### Examples

### (Mouse)

In the Experimental Examples, since transplantation of human iPS cells to mice corresponds to heterologous cell transplantation, immunodeficient male mice, i.e., NOD/Shiscid-IL2Rynull mice (hereinafter referred to as "NOG mice") are used. These species of mice are widely used in transplantation experiments of human iPS cells (K. Miura, et al. Nat Biotechnol (2009), 27: 743-5). The mice were obtained from the Central Institute for Experimental Animals.

### (Solid feed (solid food))

In the following Experimental Examples, solid feed A10021B by Research Diet was used as a control solid feed. Solid feeds, A05080209 (valine deficient feed) (Research Diet) and A05080220 (non-essential amino acid deficient feed) (Research Diet) deficient in amino acid were prepared by removing amino acids from the solid feed used as a basic feed. The energy amounts of these feeds A10021B, A05080209 and A05080220 were adjusted so as to have a same value of 3.87 kcal/g. A feed deficient in serine and glycine by Test diet (Mod TestDiet (registered trademark) 5CC7 w/ No Added Serine or Glycine, 5BJX 5CC7, 3.97 kcal/g) was used. The compositions of individual feeds are shown in the following Table.

**[Table 2]**

| Composition of solid feed (Part 1) | | | | | | |
|---|---|---|---|---|---|---|
| | A10021B | | A0508029 (valine-deficient feed) | | A05080220 (non-essential amino acid-deficient feed) | |
| | g % | kcal % | g % | kcal % | g % | kcal % |
| Protein | 17 | 18 | 16 | 17 | 7 | 7 |
| Carbohydrate | 69 | 71 | 69 | 72 | 78 | 81 |
| Lipid | 5 | 12 | 5 | 12 | 5 | 12 |
| Total | | 100 | | 100 | | 100 |
| kcal/g | | | | | | |
| L- arginine | 10 | 40 | 10 | 40 | 0 | 0 |
| L- histidine-HCl-H₂O | 6 | 24 | 6 | 24 | 6 | 24 |
| L-isoleucine | 8 | 32 | 8 | 32 | 8 | 32 |
| L-lysine | 12 | 48 | 12 | 48 | 12 | 48 |
| L-lysine-HCl | 14 | 56 | 14 | 56 | 14 | 56 |
| L-methionine | 6 | 24 | 6 | 24 | 6 | 24 |
| L-phenylalanine | 8 | 32 | 8 | 32 | 8 | 32 |
| L-threonine | 8 | 32 | 8 | 32 | 8 | 32 |
| L-tryptophane | 2 | 8 | 2 | 8 | 2 | 8 |
| L-valine | 8 | 32 | 0 | 0 | 8 | 32 |
| L-alanine | 10 | 40 | 10 | 40 | 0 | 0 |
| L-asparagine-H₂O | 5 | 20 | 5 | 20 | 0 | 0 |
| L-asparagine | 10 | 40 | 10 | 40 | 0 | 0 |
| L-cysteine | 4 | 16 | 4 | 16 | 0 | 0 |
| L-glutamic acid | 30 | 120 | 30 | 120 | 0 | 0 |
| L-glutamine | 5 | 20 | 5 | 20 | 0 | 0 |
| Glycine | 10 | 40 | 10 | 40 | 0 | 0 |
| L-proline | 5 | 20 | 5 | 20 | 0 | 0 |
| L-serine | 5 | 20 | 5 | 20 | 0 | 0 |
| L-tyrosine | 4 | 16 | 4 | 16 | 0 | 0 |
| Cornstarch | 550.5 | 2202 | 558.5 | 2234 | 648.5 | 2594 |
| Maltodextrin 10 | 125 | 500 | 125 | 500 | 125 | 500 |
| Cellulose | 50 | 0 | 50 | 0 | 50 | 0 |
| Corn oil | 50 | 450 | 50 | 450 | 50 | 450 |
| Mineral Mix S10001 *1 | 35 | 0 | 35 | 0 | 35 | 0 |
| Sodium bitartrate | 7.5 | 0 | 7.5 | 0 | 7.5 | 0 |
| Vitamin Mix V10001 *2 | 10 | 40 | 10 | 40 | 10 | 40 |
| Choline bitartrate | 2 | 0 | 2 | 0 | 2 | 0 |
| Yellow pigment FD&C #5 | 0 | 0 | 0 | 0 | 0 | 0 |
| Red pigment FD&C #40 | 0 | 0 | 0.025 | 0 | 0.05 | 0 |
| Blue pigment FD&C #1 | 0.5 | 0 | 0.025 | 0 | 0.05 | 0 |
| Total | 1000.05 | 3872 | 1000.05 | 3872 | 1000.1 | 3872 |

| | | | | | | |
|---|---|---|---|---|---|---|
| *1 Composition of Mineral Mix S10001 (weight of each component per Mix (1000 g)): calcium hydrogen phosphate (500 g), magnesium oxide (24 g), calcium citrate (220 g), potassium sulfate (52 g), sodium chloride (74 g), chromic potassium sulfate (0.55 g), copper carbonate (0.3 g), potassium iodide (0.01 g), ferric citrate (6.0 g), magnesium carbonate (3.5 g), sodium selenite (0.01 g), zinc carbonate (1.6 g) and sucrose (118.03 g). *2 Composition of Vitamin Mix V10001 (weight of each component per Mix (10 g)): vitamin A palmitate (20, 000 IU), vitamin D3 (1, 000 IU), vitamin E acetate (50 IU), menadione sodium bisulfite (0.5 mg), biotin (0.3 mg), cyanocobalamin (10 **µ** g), folic acid (6 mg), nicotinic acid (30 mg), calcium pantothenate (30 mg), pyridoxine hydrochloride (6 mg), riboflavin (6 mg), thiamine hydrochloride (6 mg), ascorbic acid (500 mg), sucrose (9.7842 g). | | | | | | |

**[Table 3-1]**

| Composition of solid feed (Part 2) | |
|---|---|
| | 5BJX 5CC7 (serine and glycinedeficient feed) |
| Component | (%) |
| Cornstarch | 41.7824 |
| Sucrose | 25.9000 |
| Baker AA Premix/No Ser or Gly (all are added) *3 | 16.0000 |
| Baker Amino Acid Mineral Premix *4 | 10.0000 |
| Corn oil | 5.0000 |
| Sodium bitartrate | 1.0000 |
| Baker Amino Acid Vitamin Premix | 0.2000 |
| Choline hydrochloride | 0.1000 |
| Ethoxyquin (preservative) | 0.0136 |
| DL-α-tocopherol acetate (vitamin E form) | 0.0040 |
| | |
| Nutrition profile | |
| Protein(%) | 14.9 |
| Arginine (%) | 0.95 |
| Histidine (%) | 0.56 |
| Isoleucine (%) | 0.91 |
| Leucine (%) | 1.37 |
| Lysine (%) | 1.26 |
| Methionine (%) | 0.69 |
| Cysteine (%) | 0.46 |
| Phenylalanine (%) | 0.91 |
| Tyrosine (%) | 0.46 |
| Threonine (%) | 0.89 |
| Tryptophane (%) | 0.23 |
| Valine (%) | 0.91 |
| Alanine (%) | 1.14 |
| Asparagine (%) | 1.14 |
| Glutamic acid (%) | 1.14 |
| Glycine (%) | 0.00 |
| Proline (%) | 1.14 |
| Serine (%) | 0.00 |
| Taurine (%) | 0.00 |

**[Table 3-2]**

| Composition of solid feed (Part 2, continued) | |
|---|---|
| Lipid (%) | 5.1 |
| Cholesterol (ppm) | 0 |
| Linoleic acid | 2.86 |
| Linolenic acid | 0.05 |
| Arachidonic acid | 0.00 |
| ω3 Fatty acid | 0.05 |
| Total saturated fatty acid | 0.64 |
| Total monounsaturated fatty acid | 1.21 |
| Total polyunsaturated fatty acid | 2.90 |
| Fiber (maximum) (%) | 0.0 |
| | |
| Carbohydrate (%) | 72.8 |
| | |
| Energy (kcal/g) | 3.97 |
| Derived from protein | 0.597 kcal, 15.0% |
| Derived from lipid (extracted with ether) | 0.458 kcal, 11.6% |
| Derived from carbohydrate | 2.911 kcal, 73.4% |
| | |

| Minerals | |
|---|---|
| Calcium (%) | 1.21 |
| Phosphate (%) | 0.72 |
| Potassium (%) | 0.41 |
| Magnesium (%) | 0.01 |
| Sodium (%) | 0.64 |
| Chlorine (%) | 1.23 |
| Fluorine (ppm) | 0.0 |
| Iron (ppm) | 87 |
| Zinc (ppm) | 52 |
| Magnesium (ppm) | 211 |
| Copper (ppm) | 5.0 |
| Cobalt (ppm) | 0.3 |
| Iodonium (ppm) | 30.58 |
| Chromium (added) (ppm) | 0.0 |
| Molybdenum (ppm) | 35.69 |
| Selenium (ppm) | 0.46 |

**[Table 3-3]**

| Composition of solid feed (Part 2, continued) | |
|---|---|
| Vitamin | |
| Vitamin A (IU/g) | 5.2 |
| Vitamin D3 (added) (IU/g) | 0.9 |
| Vitamin E (IU/kg) | 20.0 |
| Vitamin K (ppm) | 2.00 |
| Thiamine hydrochloride (ppm) | 18.4 |
| Riboflavin (ppm) | 10.0 |
| Niacin (ppm) | 50 |
| Pantothenic acid (ppm) | 28 |
| Folic acid (ppm) | 4.0 |
| Pyridoxine (ppm) | 4.9 |
| Biotin (ppm) | 0.6 |
| Vitamin B12 (mcg/kg) | .38 |
| Choline hydrochloride (ppm) | 700 |
| Ascorbic acid (ppm) | 250.0 |

| | |
|---|---|
| *3 Components of Baker Amino Acid Vitamin Premix: sucrose, ascorbic acid, inositol, nicotinic acid, calcium pantothenate, thiamine nitrate, riboflavin, pyridoxine hydrochloride, vitamin A acetate, folic acid, vitamin B12, menadione sodium bisulfite (vitamin K supply source), aminobenzoic acid, cholecalciferol and biotin. *4 Components of Baker Amino Acid Mineral Premix: cornstarch, calcium phosphate, potassium phosphate, sodium chloride, calcium carbonate, magnesium sulfate, iron citrate, magnesium sulfate, zinc carbonate, sodium molybdate, boric acid, potassium iodide, copper sulfate, sodium selenite and cobalt sulfate. *5 Baker AA Premix/No Ser or Gly: L-lysine-hydrochloride, L-leucine, L-arginine-HCl, L-alanine, L-asparagine, glutamic acid, L-glutamine, L-proline, L-phenylalanine, L-valine, L-threonine, L-isoleucine, L-methionine, L-histidine-HCl-H₂O, L-tyrosine, L-cysteine and L-tryptophan. | |

### [Experimental Example 1]

Transplantation Experiment 1: Valine deficient feed

NOG mice of 6-weeks old were delivered, acclimated for a week by feeding and used for experiments. After the body weights were measured, the mice were anesthetized by inhalation of 1.5-2.0% isoflurane. Under anesthesia, opening was made from the right center or left center of the back and the kidney was exposed. Human iPS cells 1383D2 strain (obtained from the Center for iPS Cell Research and Application, Kyoto University) (one million cells) were transplanted under the renicapsule by use of an injection needle. Eighteen mice in total were transplanted with the iPS cells. Four mice, which were subjected to the same surgical operation but not subjected to transplantation, were used as a Sham group. Thereafter, the kidney was placed again in the abdomen and the opening was surgically closed. After recovery from anesthesia was confirmed, the mice were placed in a cage (3 mice/cage, 2 mice/cage only in the case of Sham group). Thereafter, a transplant group of 18 mice were divided into a control feed group (6 mice) and 2 valine deficient feed groups (6 mice × 2). To a Sham group (4 mice), a valine deficient feed was fed. The feed was weekly exchanged with new one and the body weight was measured on the third week. To recover weight loss, the feed for the 2 valine deficient feed groups (6 mice × 2) was changed to a control feed. Next week (on the fourth week after transplantation), the feed for one (6 mice) of the valine deficient feed groups was changed again to a valine deficient feed. Thereafter, to this group, the valine deficient feed and the control feed were alternately fed week by week, until the end of experiment. The other valine deficient group (6 mice), to which the control feed was started to be fed from three weeks ago, was continuously fed with the control feed until the end of the experiment. On the 68th day after transplantation, the mice were dissected under anesthesia. The weights of the kidney having cells transplanted and the opposite-side kidney having no cells transplanted were both measured and the difference between them were calculated and regarded as the weight of teratoma.

**[Table 4]**

| Transplantation Experiment 1 (valine deficient feed) experiment group/control group | | | |
|---|---|---|---|
| Group # | Mouse | Conditions | n |
| 1 | iPS (1383D2) | Feeding of control feed (CTL) was continued | 6 |
| 2 | iPS (1383D2) | Feeding of valine-deficient feed (Va (-)) for 3 weeks and feeding of control feed (CTL) for 3 weeks were repeated alternately | 6 |
| 3 | iPS (1383D2) | Feeding of valine-deficient feed (Va (-)) for 3 weeks, and then, feeding of control feed (CTL) was continued | 6 |
| 4 | iPS (1383D2) Sham | Feeding of valine-deficient feed (Va (-)) for 3 weeks and feeding of control feed (CTL) for 3 weeks were repeated alternately | 4 |

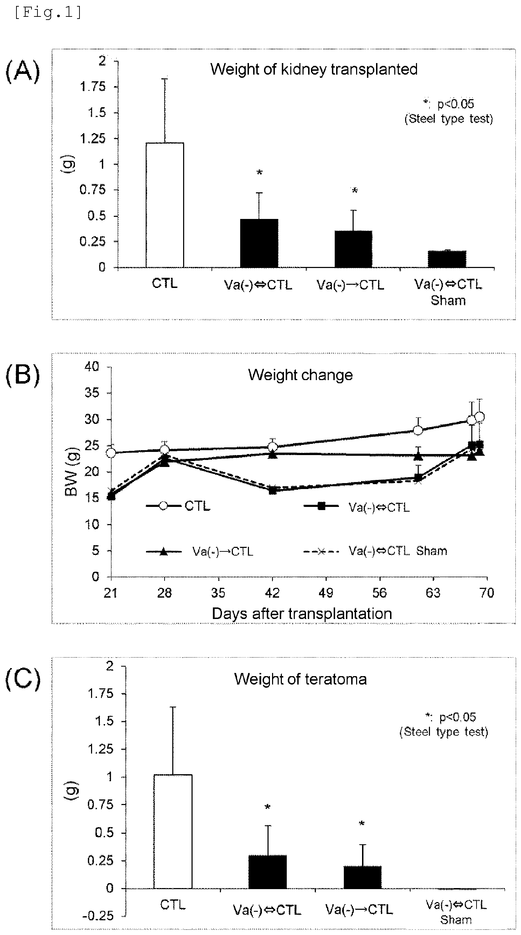

The results of teratoma weight and others in Transplantation Experiment 1 are shown in Figure 1. A significant suppressive effect by the valine deficient feed on formation of teratoma was confirmed.

### [Experimental Example 2 (reference)]

### Transplantation Experiment 2: Serine and glycine deficient feed

NOG mice of 6-weeks old were delivered, acclimated for a week by feeding and used for experiments. After the body weights were measured, the mice were anesthetized by inhalation of 1.5-2.0% isoflurane. Under anesthesia, opening was made from the right center or left center of the back and the kidney was exposed. Human iPS cells 1383D2 strain (obtained from the Center for iPS Cell Research and Application, Kyoto University) (five million cells) were transplanted under the renicapsule by use of an injection needle. Eighteen mice in total were transplanted with the iPS cells. Four mice, which were subjected to the same surgical operation but no transplantation was carried out, were used as a Sham group. Thereafter, the kidney was placed again in the abdomen and the opening was surgically closed. After recovery from anesthesia was confirmed, the mice were placed in a cage (3-4 mice/cage, 2-4 mice/cage only in the case of Sham group). Thereafter, a transplant group of 20 mice were divided into a control feed group (10 mice) and a serine/glycine deficient feed group (10 mice). To a Sham group (5 mice), a serine and glycine deficient feed was fed. The feed was weekly exchanged with new one and the body weight was measured on Day 7, 14, 28 and 48 after transplantation. The mice were dissected under anesthesia on Day 48 after transplantation. The weights of the kidney having cells transplanted and the opposite-side kidney having no cells transplanted were both measured and the difference between them were calculated and regarded as the weight of teratoma.

**[Table 5]**

| Transplantation Experiment 2 (serine/glycine deficient feed) experiment group/control group | | | |
|---|---|---|---|
| Group # | Mouse | Conditions | n |
| 1 | iPS (1383D2) | Feeding of control feed (CTL) was continued | 10 |
| 2 | iPS (1383D2) | Feeding of serine/glycinedeficient feed was continued | 10 |
| 3 | iPS (1383D2) Sham | Feeding of serine/glycinedeficient feed was continued | 5 |

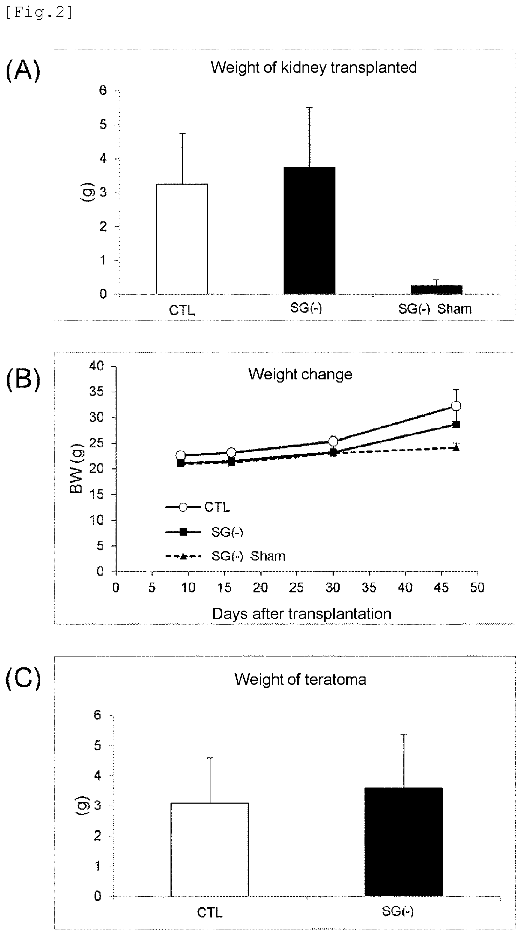

The results of teratoma weight and others in Transplantation Experiment 2 are shown in Figure 2. It was not confirmed that the serine/glycine deficient feed has a significant suppressive effect on formation of teratoma.

### [Experimental Example 3 (reference)]

### Transplantation Experiment 3: Non-essential amino acid deficient feed

NOG mice of 8-weeks old were delivered, acclimated for a week by feeding and used for experiments. After the body weights were measured, the mice were anesthetized by inhalation of 1.5-2.0% isoflurane. Under anesthesia, opening was made from the right center or left center of the back and the kidney was exposed. Human iPS cells 1383D2 strain (obtained from the Center for iPS Cell Research and Application, Kyoto University) (five million cells) were transplanted under the renicapsule by use of an injection needle. Fifteen mice in total were transplanted with the iPS cells. Thereafter, the kidney was placed again in the abdomen and the opening was surgically closed. After recovery from anesthesia was confirmed, the mice were placed in a cage (2-4 mice/cage). Thereafter, a transplant group of 15 mice were divided into a control feed group (5 mice) and a non-essential amino acid (asparagine, aspartic acid, alanine, arginine, glycine, glutamine, glutamic acid, cysteine, serine, tyrosine and proline) deficient feed group (10 mice). The feed was weekly exchanged with new one and the body weight was measured on Day 3, 10, 24, 43 and 50 after transplantation. The mice were dissected under anesthesia on Day 50 after transplantation. The weights of the kidney having cells transplanted and the opposite-side kidney having no cells transplanted were both measured and the difference between them were calculated and regarded as the weight of teratoma.

**[Table 6]**

| Transplantation Experiment 3 (non-essential amino acid deficient feed) experiment group/control group | | | |
|---|---|---|---|
| Group # | Mouse | Conditions | n |
| 1 | iPS (1383D2) | Feeding of control feed (CTL) was continued | 5 |
| 2 | iPS (1383D2) | Feeding of non-essential amino acid-deficient feed (NEAA(-)) was continued | 10 |

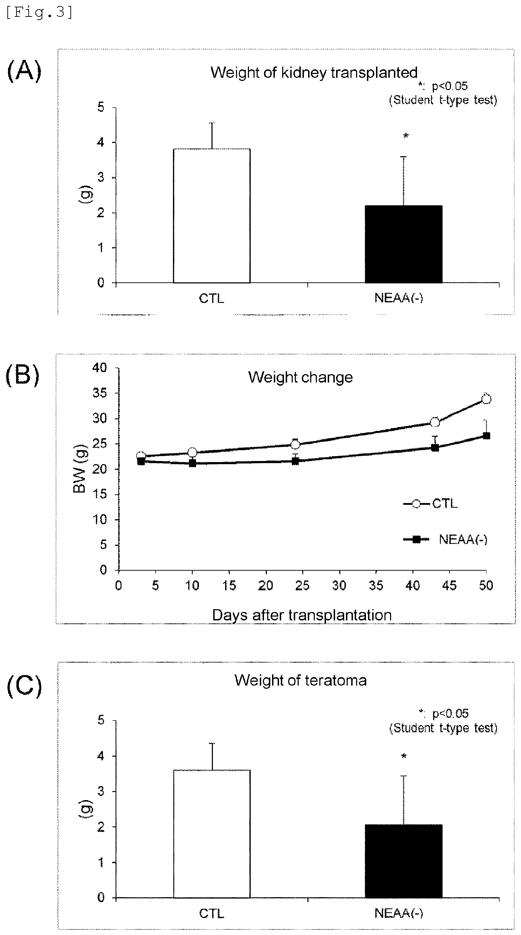

The results of teratoma weight and others in Transplantation Experiment 3 are shown in Figure 3. It was confirmed that the non-essential amino acid deficient feed has a significant suppressive effect on formation of teratoma.

### [Experimental Example 4]

### Verification of survival-rate reduction of stem cells in valine-free medium

### (1) Culture of human iPS cells

Human iPS cells (1383D2; the Center for iPS Cell Research and Application, Kyoto University) were seeded in StemFit AK02N on the coating of Laminin-511. One day later, the medium was exchanged with the following medium. Two days after medium exchange, a cell survival rate was determined by Cells-Titer Glo (Promega).

Valine-containing medium: DMEM/F-12 (Gibco), E8 supplement (Thermo).

Valine-free medium: DMEM/F-12 (-Val) (Research Institute for the Functional Peptides Co., Ltd, custom order), E8 supplement (Thermo).

### (2) Results

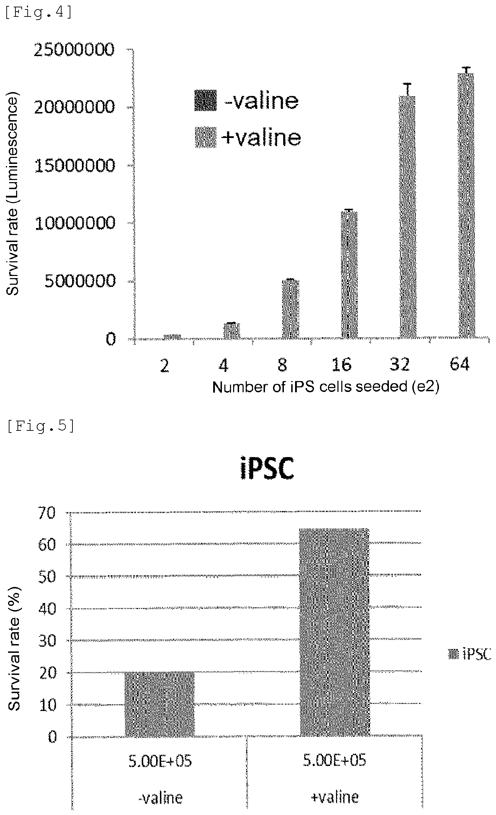

The results are shown in Figure 4. Virtually no survival cells of human iPS cells cultured in a valine-free medium were found two day after medium exchange.

### [Experimental Example 5]

### Verification of survival-rate reduction of stem cells mixed in liver organoid in valine-free medium

### (1) Preparation of human endothelial cells (EC)

Human iPS cells (1383D2; the Center for iPS Cell Research and Application, Kyoto University) were cultured in the medium, which was prepared by adding, to DMEM/F-12 (Gibco) (10 ml), 1% B-27 Supplements (GIBCO), BMP4 (25 ng/ml) and CHIR99021 (8 µM), in the conditions of a 5% CO₂ and 37°C for 3 days to induce mesodermal cells. The mesodermal cells obtained were further cultured in a medium, which was prepared by adding, to Stempro-34 SFM (Gibco) (10 ml), VEGF (200 ng/ml) and Folskolin (2µM), in the conditions of 5% CO₂ and 37°C for 7 days to obtain CD31-positive, a CD73-positive and CD144-positive human non-hematopoietic vascular endothelial cell population.

### (2) Preparation of human hepatic endoderm cells (HE)

Human iPS cells (1383D2) were cultured in RPMI 1640 (FUJIFILM) (2 ml), to which Wnt3a (50 ng/ml) and activin A (100 ng/ml) were added, in the conditions of 5% CO₂ and 37°C for 5 days to induce endodermal cells. The endodermal cells obtained were further cultured in the same medium, to which 1% B27 Supplements (GIBCO) and FGF2 (10 ng/ml) were added, in the conditions of 5% CO₂ and 37°C for 5 days to obtain an AFP-, ALB- and HNF4α-positive human hepatic endoderm cell population.

### (3) Preparation of human mesenchymal stem cells (MC)

Human iPS cells (1383D2) were cultured in DMEM/F-12 (Gibco) (10 ml), to which 1% B-27 Supplement (GIBCO), BMP4 (25 ng/ml) and CHIR99021 (8 µM) were added, in the conditions of 5% CO₂ and 37°C for 3 days to induce mesodermal cells. The mesodermal cells obtained were cultured in the same medium, to which PDGFBB (10 ng/ml) and activin A (2 ng/ml) were added, in the conditions of 5% CO₂ and 37°C for 3 days, and thereafter, further cultured in DMEM/F-12 (Gibco) (10 ml), to which 1% B-27 Supplements (GIBCO), FGF2 (10 ng/ml) and BMP4 (12 ng/ml) were added, in the conditions of 5% CO₂ and 37°C for 3 days to obtain human mesenchymal stem cells.

### (4) Preparation of organoid (three-dimensional structure) and co-culture with human iPS cells

The human hepatic endoderm cells (HE), human vascular endothelial cells (EC), human mesenchymal stem cells (MC) and human iPS cells (1383D2) were mixed in a ratio of 10: 7: 1: 5 (total number of cells: 2.3 × 10⁶) and co-cultured in a 3d culture vessel, Elplasia (Kuraray Co., Ltd), for one day in the conditions of 5% CO₂ and 37°C to produce aggregates. The culture medium (herein, referred to as "organoid medium (A)") used in the co-culture was prepared by blending a medium for hepatocytes (A), which was prepared by adding, to HCM (Lonza), FBS (5%), HGF (10 ng/ml), OSM (20 ng/ml) and Dex (100 nM), and a medium for vascular endothelial cells (A), which was prepared by adding, to Stempro-34 SFM (Gibco), VEGF (50 ng/ml) and FGF2 (10 ng/ml), in a volume ratio of 1: 1. One day after co-culture, the organoid medium (A) was exchanged with the following medium. One day after medium exchange, the survival rate of cells was determined by FACS fortessa.

Valine-containing medium: a mixture of a medium for hepatocytes (B), which was prepared by adding, to DMEM/F-12 (Gibco), KSR (5%), HGF (10 ng/ml), OSM (20 ng/ml) and Dex (100nM), and the above medium for vascular endothelial cells (A) in a volume ratio of 1: 1.

Valine-free medium: a mixture of medium for hepatocytes (B'), which was prepared by adding, to DMEM/F-12 (-Val) (Research Institute for the Functional Peptides Co., Ltd., custom order) KSR (5%), HGF (10 ng/ml), OSM (20 ng/ml) and Dex (100 nM), and the above medium for vascular endothelial cells (A) in a volume ratio of 1: 1.

### (5) Results

The results are shown in Figure 5. The number of human iPS cells cultured together with organoid in the valine-free medium decreased one day after the medium exchange up to 1/3 compared to that in the valine-containing medium.

## Claims

1. A nutrition composition for suppressing formation and/or proliferation of undesired cells derived from stem cells in a cell population containing cells differentiated from stem cells, the nutrition composition comprising at least one essential amino acid selected from the group consisting of isoleucine, leucine, methionine, lysine, phenylalanine, tryptophan, threonine and histidine except valine, and optionally comprising a non-essential amino acid(s).

2. The nutrition composition according to claim 1, wherein the nutrition composition comprises at least methionine as the essential amino acid.

3. The nutrition composition according to claim 1, wherein the nutrition composition comprises no non-essential amino acid.

4. The nutrition composition according to claim 1, wherein the nutrition composition comprises at least one non-essential amino acid selected from the group consisting of arginine, glycine, serine, asparagine and glutamine.

5. The nutrition composition according to claim 1, wherein the nutrition composition further comprises a nutrient other than the amino acids.

6. The nutrition composition according to claim 1, wherein the nutrition composition is to be taken for 11 days or more.

7. The nutrition composition according to claim 1, wherein the nutrition composition is 1) selected from a solid food, a solid agent, a semi-solid food, a semi-solid agent, a beverage, and a liquid, or is 2) a culture medium.

8. A kit comprising: the nutrition composition according to claim 1; and a cell population containing cells differentiated from stem cells.

9. A method for suppressing formation and/or proliferation of undesired cells derived from stem cells, comprising allowing a cell population containing cells differentiated from stem cells to take the nutrition composition according to claim 1.

10. Use of the nutrition composition according to claim 1 for suppressing formation and/or proliferation of undesired cells derived from stem cells in a cell population containing cells differentiated from stem cells.

11. A method for suppressing formation and/or proliferation of undesired cells derived from stem cells *in vivo,* comprising allowing a mammal, to which a cell population containing cells differentiated from stem cells has been transplanted or administered, to take the nutrition composition according to claim 1.
